**Europäisches Patentamt**

**European Patent Office**

**Office européen des brevets**

(19)

(11) Veröffentlichungsnummer: **0 250 863 B1**

# EUROPÄISCHE PATENTSCHRIFT

(12)

(45) Veröffentlichungstag der Patentschrift: **29.04.92**

(51) Int. Cl.⁵: **A61F 7/00**, F25D 3/10

(21) Anmeldenummer: **87107575.0**

(22) Anmeldetag: **25.05.87**

(54) Vorrichtung zur Erzeugung eines kalten Behandlungsgases für die Kryotherapie.

(30) Priorität: **25.06.86 DE 3621425**

(43) Veröffentlichungstag der Anmeldung:
**07.01.88 Patentblatt 88/01**

(45) Bekanntmachung des Hinweises auf die
Patenterteilung:
**29.04.92 Patentblatt 92/18**

(84) Benannte Vertragsstaaten:
**AT BE CH FR GB IT LI NL SE**

(56) Entgegenhaltungen:
**EP-A- 0 175 047**
**DE-A- 2 025 224**
**DE-A- 2 613 000**
**FR-A- 2 242 062**
**US-A- 3 823 575**

**BIOMEDIZINISCHE TECHNIK, Band 27, Nr. 11,**
**November 1982, Seite 288, Berlin, DE;**
**"Entfernen von Warzen mit Flüssigstickstoff**
**schnell, sauber, feinfühlig"**

(73) Patentinhaber: **MESSER GRIESHEIM GMBH**
**Hanauer Landstrasse 330**
**W-6000 Frankfurt/Main(DE)**

(72) Erfinder: **Donnerhack, Andreas, Dr.**
**Bismarckstrasse 4**
**W-4150 Krefeld(DE)**
Erfinder: **Pfeil-Schneider, Kurt**
**Johannesfeld 29**
**W-4054 Nettetal(DE)**

EP 0 250 863 B1

**Beschreibung**

Die Erfindung betrifft eine Vorrichtung zur Erzeugung eines kalten Behandlungsgases aus flüssigem Stickstoff für die Kryotherapie nach dem Oberbegriff des Anspruches 1.

Vorrichtungen zur Durchführung der Kryotherapie mittels eines kalten Behandlungsgases, welches aus flüssigem Stickstoff gewonnen wird, sind bekannt. In der Praxis resultieren daraus bislang nur recht aufwendige Geräte. Diese werden dem geforderten hohen Standard in Kliniken oder größeren Praxen durchaus gerecht. Für Ärzte, die die Kryotherapie nur bei sehr wenigen Patienten anwenden, stehen die hohen Investitionskosten der Einführung derartiger Geräte zur Kaltwinderzeugung bislang häufig entgegen. Im Bereich der niedergelassenen Ärzte besteht daher ein Bedarf für ein vereinfachtes Kaltwindgerät, mit dem die bislang bekannten Behandlungskosten erheblich reduziert werden können. Dies trifft auch zu auf einzelne Patienten, die die Kryotherapie bei sich zu Hause durchführen. Es ist bekannt, daß sich Patienten nach entsprechender Diagnosestellung und Anweisung durch den Arzt selbst mit Kälte behandeln. Die Anwendung von Kältepackungen ist bei sorgfältiger Durchführung auch für den Patienten unproblematisch. Da die Kältepackungen über einen längeren Zeitraum kontinuierlich anzuwenden sind, ist die Selbstbehandlung in solchen Fällen sinnvoll und sogar wünschenswert. Die gleichen Voraussetzungen und Randbedingungen gelten auch für die Behandlung mit einem kalten Behandlungsgas. Für die Selbstbehandlung steht jedoch bisher kein geeignetes Gerät zur Verfügung.

Ein Therapiegerät, das in den beiden genannten Bereichen eingesetzt werden kann, muß zwei Anforderungen genügen. Zum einen müssen die Investionskosten in einem Bereich liegen, der auch noch für einen Privatmann tragbar ist. Zum anderen dürfen sich jedoch aus der Vereinfachung des Gerätes keine Sicherheitsrisiken für den Anwender ergeben. Denn besonders für den Bereich der Selbstbehandlung muß der Tatsache Rechnung getragen werden, daß der Betreiber in der Regel technischer und medizinischer Laie ist. Der Erfindung liegt daher die Aufgabe zugrunde, eine Vorrichtung zur Erzeugung eines kalten Behandlungsgases aus flüssigem Stickstoff für die Kryotherapie zu schaffen, welches nur für gelegentlichen Einsatz konzipiert ist und dabei einfach im Aufbau, preiswert in der Herstellung und unkompliziert in der Bedienung ist, gleichzeitig aber auch kein Sicherheitsrisiko für den Anwender darstellt.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben. Ein wesentliches Merkmal der erfindungsgemäßen Vorrichtung ist das fest in den Flüssigstickstoff-Behälter eingebaute Heizelement. Dieses als selbstregulierendes Heizband ausgebildete Heizkabel kann am Boden des Behälters durch dort angebrachte Klammern spiralenförmig befestigt werden. Die Zuleitung kann durch entsprechende Klammern an der inneren Behälterwandung nach oben und durch den Behälterhals hinausgeführt werden. Ein solches Heizelement kann in der genannten Art bereits vor der Endmontage des Behälters befestigt werden, ohne daß die weiteren Arbeitsläufe bei der Behältermontage behindert werden. Bisher bekannte Geräte verwenden Heizelemente, die beim Befüllen des Behälters entfernt werden müssen. Ein bekanntes System arbeitet zwar mit einem fest in dem Flüssigstickstoff-Behälter eingebauten Heizelement. Jedoch ist dazu ein Weithalsgefäß in Sonderanfertigung notwendig. Der Vorteil der erfindungsgemäßen Vorrichtung besteht darin, daß zum einen auf Standardbehälter zurückgegriffen werden kann, zum anderen trotz des festen Einbaus der Heizung die geringe Verdampfungsrate von Enghalsbehältern genutzt werden kann.

Wesentlich zur Verwirklichung der angestrebten Vereinfachung ist die Ausbildung des Heizelementes als selbstregulierendes Heizband.

Um Beschädigungen am Heizelement bzw. am Behälter zu vermeiden, wenn der flüssige Stickstoff im Behälter verbraucht ist, müssen bei den bekannten Systemen aufwendige Temperatur- bzw. Heizstrom-Regelvorrichtungen vorgesehen werden. Die Verwendung eines selbstregulierenden Heizbandes macht diese Vorrichtungen überflüssig. Diese Heizkabel sind im Handel erhältlich. Für die erfindungsgemäße Vorrichtung wird die Tatsache ausgenutzt,daß ohne externe Beeinflussung der Stromzufuhr bei Erwärmung die Heizleistung abfällt und eine bestimmte Grenztemperatur nicht überschritten wird. Durch richtige Dimensionierung des selbstregulierenden Heizbandes kann die maximale Grenztemperatur so festgelegt werden, daß bei fehlendem Flüssigstickstoff weder Behälter noch Heizelement beschädigt werden. Das selbstregulierende Heizband ist mit einer Isolationsschicht aus Kunststoff versehen, die eine hohe Bruchstabilität unter tiefen Temperaturen aufweist. Diese Isolationsschicht wird daher bei der bestimmungsgemäßen Verwendung im Flüssigstickstoff-Behälter mechanisch nicht beeinträchtigt. Darüber hinaus bildet diese Kunststoffschicht einen Schutz der stromfüh-

2

renden Leiter vor Feuchtigkeitszutritt. Die immer in Verbindung mit tiefen Temperaturen zu erwartende Feuchtigkeitführt daher nicht zu einer Beeinträchtigung der elektrischen Sicherheit des Systems.

Unter Berücksichtigung des vorgesehenen Einsatzzweckes wird das Behältervolumen auf maximal 50 1 beschränkt. Die erfindungsgemäße Vorrichtung kann daher platzsparend aufgestellt werden. Andererseits kann sie mit einer Befüllung mehrere Wochen betrieben werden.

Als Behälter für den flüssigen Stickstoff kann daher ein serienmäßiges Gefäß mit Kleinflansch, das üblicherweise mit Sicherheitsventil und Manometer ausgerüstet ist, verwendet werden. Für den Einsatz im Sinne der Erfindung ist ein Druckaufbau durch den Wärmeeintrag nur insofern erforderlich, als das entstehende Kaltgas aus dem nach oben offenen Gefäß ausgetrieben werden muß. Auf das Manometer kann daher verzichtet werden. Statt dessen wird durch die dafür vorgesehene seitliche Öffnung am Hals die Zuleitung des Heizkabels herausgeführt. Als Zuleitungskabel werden dazu bekannte Elektrokabel verwendet, deren Isolationsschichten aus kältebeständigem Kunststoff bestehen. Diese Leitungen sind ebenfalls so montiert, daß sie nicht bewegt werden können. Eine mechanische Beanspruchung unter Kälte erfolgt daher nicht. Das Sicherheitsventil verbleibt am Behälter, so daß das System gegen einen unerwünschten Druckaufbau durch einen fahrlässigen oder unbeabsichtigten Verschluß der Ausblasöffnung gesichert ist.

Bei dem beschriebenen System ist die eigentliche Halsöffnung im Flanschbereich vollkommen frei von der Heizvorrichtung. Das Anbringen des Behandlungsschlauches kann daher im Vergleich zu den bekannten Geräten wesentlich vereinfacht werden, da bei einer Demontage zum Befüllen des Behälters nur der eigentliche Behandlungsschlauch, nicht aber das Heizelement entfernt werden muß.

Das Abkühlen der äußeren Schlauchoberflächen muß vermieden werden, jedoch nicht für Dauerbetrieb sondern nur für eine Einzelbehandlung, die in der Regel nicht länger als 2 bis 3 Minuten dauert. Es kann daher auf eine aufwendige Isolation des Schlauches verzichtet werden. Es hat sich als ausreichend erwiesen, den eigentlichen kaltgasführenden Spiralschlauch aus PVC lediglich mit je einer Schicht aus Kunststoffolie und folienbeschichtetem Filz zu isolieren. Darüber wird ein umhüllender Kunststoffwellenschlauch gezogen.

Verzichtet wird bei der erfindungsgemäßen Vorrichtung auf die Temperaturregelung des Kaltgases. Durch die stromkonstante Heizung wird während des Betriebes ein konstanter Kaltgasstrom gefördert, der nach kurzer Anlaufzeit auch eine konstante Kaltgastemperatur am Schlauchaustritt zur Folge hat.

Unter den genannten Voraussetzungen kann damit auch die elektrische Steuerung des Gerätes im Vergleich zu den bisher bekannten Systemen erheblich vereinfacht werden. Das Heizband kann direkt an die Netzspannung angeschlossen werden. Um den erforderlichen Sicherheitskriterien zu genügen, ist lediglich ein Hauptschalter und ein Sicherungselement in diesen Stromkreislauf einzufügen.

Zusätzlich kann dieser Stromkreis mit einer Zeitschaltuhr, mit der sich bei Inbetriebnahme die Behandlungszeit vorwählen läßt, versehen werden. Nach Ablauf dieser Uhr schaltet das Gerät selbsttätig aus. Diese Zeitschaltuhr ist für den Betrieb des Therapiegerätes nicht zwingend erforderlich; sie erhöht jedoch den Bedienungskomfort, ohne dem Ziel der Erfindung eines preiswerten Gerätes entgegenzustehen.

Die genannten elektrischen Installationselemente können in einem kleinen Gehäuse am Behälterhals befestigt werden, ohne daß die Demontage des Behandlungsschlauches sowie das Befüllen des Behälters durch die Halsöffnung behindert werden.

Die Zeichnungen veranschaulichen ein Ausführungsbeispiel der Erfindung.

Es zeigen:

Fig.1      eine aufgebrochene Seitenansicht eines Behälters mit Heizelement,

Fig.2      den Behälterhals.

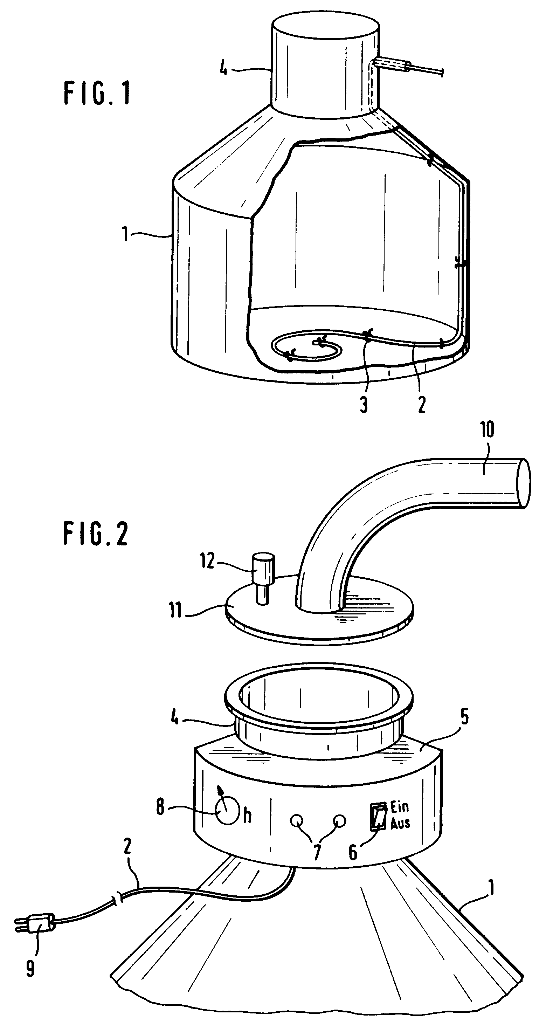

Der in Fig.1 dargestellteBehälter 1 ist ein Standardgefäß in Enghalsausführung mit 30 1 Rauminhalt. Einzelheiten der Isolierung und doppelwandigen Ausführung sind nicht dargestellt. Erfindungsgemäß ist am Boden und an der Behälterinnenwand ein selbstregulierendes Heizband 2 mit Hilfe von Klammern 3 fest installiert und durch den Behälterhals 4 nach außen geführt.

Fig.2 zeigt den Behälterhals im Detail. An ihm ist ein Gehäuse 5 befestigt, in welchem der Hauptschalter 6, Sicherungen 7 und eine Zeitschaltuhr 8 angeordnet sind. Sobald der Stecker 9 an das elektrische Netz angeschlossen ist, ist das Gerät betriebsbereit. Nach Betätigen der Zeitschaltuhr 9 wird entsprechend der vorgegebenen Zeit kaltes Behandlungsgas erzeugt, welches durch den Schlauch 10 dem Behandlungsort zugeführt wird. Der Schlauch 10 ist mittels des Flansches 11 auf dem Behälterhals 4 befestigt. Im Flansch 11 ist ein Sicherheitsventil 12 angeordnet.

Sicherheitstechnisch genügt die erfindungsgemäße Vorrichtung den Anforderungen, die für die Kaltwindtherapie gültig sind. Selbst bei kontinuierlichem Betrieb des Gerätes werden nur begrenzte Sickstoffmengen freigesetzt, so daß unter normalen Bedingungen der Sauerstoffgehalt der Raumluft nicht deutlich reduziert wird. Das erzeugte kalte Behandlungsgas ist trocken. Der Austritt von Flüssigstickstoff aus dem Behandlungsschlauch ist nicht möglich. Der Einsatz des Gerätes im Bereich der Selbstbehandlung ist deshalb durchaus vertretbar.

Die erfindungsgemäße Vorrichtung kann mit einem Bruchteil des Aufwandes fertiggestellt werden, der für die heute bekannten Geräte zur Durchführung der Kryotherapie notwendig ist. Die resultierenden Investitionskosten für den Patienten liegen in einer Größenordnung, die ihm die Anschaffung des Gerätes auch ohne Kostenbeteiligung durch die Krankenkasse ermöglichen kann.

Die Betriebskosten des Gerätes lassen anhand statistischer Mittelwerte für die Behandlungszeiten abschätzen. Geht man von einer durchschnittlichen Behandlungsdauer von 2 Minuten und von 2 Behandlungen pro Tag aus, so resultieren aus dem Flüssigstickstoff-Verbrauch durch diese Behandlung und der Eigenverdampfung des 30 l Gefäßes Haltezeiten des gesamten Systems von mehr als 3 Wochen. Daraus ergeben sich praktikable Betankungsintervalle durch den Kälteservice.

## Patentansprüche

1. Vorrichtung zur Erzeugung eines kalten Behandlungsgases aus flüssigem Stickstoff für die Kryotherapie, mit einem Behälter für die Speicherung des flüssigen Stickstoffs und einer im Behälterinnern angeordneten elektrischen Heizung,
dadurch gekennzeichnet,
daß der Behälter (1) ein Kleinbehälter mit maximal 50 l Fassungsvermögen ist und die elektrische Heizung ein im Behälterinnern befestigtes Heizelement aus selbstregulierendem Heizband (2) ist.

2. Vorrichtung nach Anspruch 1,
gekennzeichnet durch ein am Hals (4) des Behälters befestigtes Gehäuse (5) zur Aufnahme eines Hauptschalters (6) und einer Sicherung (7) für die Stromversorgung.

3. Vorrichtung nach Anspruch 2,
gekennzeichnet durch eine Zeitschaltuhr (8) im Gehäuse zur Vorwahl der Therapiezeit und selbsttätigen Ausschaltung der Vorrichtung.

## Claims

1. Device for producing a cold treatment gas from liquid nitrogen for cryotherapy, with a container for storing the liquid nitrogen, and with an electrical heating system arranged in the inside of the container, characterised in that the container (1) is a small container with a maximum capacity of 50 l, and the electrical heating system is a heating element, consisting of a self-regulating heater band (2), secured in the inside of the container.

2. Device according to Claim 1, characterised by a housing (5), secured on the neck (4) of the container, for accommodating a main switch (6) and a fuse (7) for the current supply.

3. Device according to Claim 2, characterised by a time switch clock (8) in the housing for preselecting the therapy time and for switching the device off automatically.

## Revendications

1. Dispositif de production de gaz froids pour le traitement par la cryothérapie, comprenant un réservoir pour stocker l'azote liquide et un moyen de chauffage électrique placé dans le réservoir,
dispositif caractérisé en ce que :
   - le réservoir (1) est un petit réservoir ayant au maximum 50 litres de capacité et le chauffage électrique est un élément chauffant fixé dans le réservoir et qui est constitué par un ruban chauffant (2) autorégulé.

2. Dispositif selon la revendication 1, caractérisé par un boîtier (5) fixé au col (4) du réservoir pour recevoir un interrupteur principal (6) et un fusible (7) pour l'alimentation électrique.

3. Dispositif selon la revendication 2, caractérisé par une horloge (8) prévue dans le boîtier pour sélectionner la durée de mise en oeuvre et assurant la coupure automatique du dispositif.

FIG. 1

FIG. 2